# DEMANDE DE BREVET EUROPEEN

(11) **EP 2 499 958 A1**
(43) Date de publication de la demande: **19.09.2012**
(21) Numéro de dépôt: 12159507.8
(22) Date de dépôt: 14.03.2012
(51) Int. Cl.: A61B 1/05, A61B 1/06, G02B 23/24, G02B 23/26

(54) **Tête optique bispectrale à usage unique pour videoéndoscope et videoendoscope associe**

(30) Priorité: 15.03.2011 FR 1152100
(71) Demandeur: Commissariat à l'Énergie Atomique et aux Énergies Alternatives, 75015 Paris (FR)
(72) Inventeur: Peltie, Philippe, 38760 SAINT PAUL DE VARCES (FR); Bouter, Jérôme, 38640 CLAIX (FR); Rizo, Philippe, 38700 LA TRONCHE (FR)
(74) Mandataire: Bréda, Jean-Marc

(57) **Abrégé**

Le domaine général de l'invention est celui des vidéoendoscopes pour usage médical ou industriel. Le vidéoendoscope selon l'invention possède une tête optique bispectrale (1) comprenant une microcaméra (3, 11), des moyens d'éclairage en lumière blanche (40) et en lumière de fluorescence (41). La tête optique est reliée par des connecteurs électriques et optiques (31, 32, 33) appropriés aux autres éléments du vidéoendoscope (21, 22, 23). Préférentiellement, elle comporte des éléments standards à bas coût et est conditionnée dans un emballage stérile à usage unique. II existe différents modes de réalisation possibles selon que la tête optique comporte un seul capteur ou deux capteurs, selon que le dispositif fonctionne en mode continu ou en mode séquentiel et selon enfin, que les sources de lumière sont incorporées dans la tête optique ou externes, la lumière étant acheminée alors au moyen de fibres optiques.

## Description

Le domaine technique de l'invention est celui de l'endoscopie, plus précisément celui des « vidéoendoscopes ». L'application préférée de ces instruments est le domaine médical, mais l'endoscopie a aussi des applications en milieu industriel. Ainsi, dans les domaines automobile ou aéronautique, on utilise de telles sondes pour l'inspection de zones non facilement accessibles comme certaines parties de moteurs.

Actuellement, les systèmes les plus utilisés en endoscopie sont soit les endoscopes rigides soit les endoscopes souples ou fibroscopes.

Les endoscopes rigides ont un diamètre compris entre 5 et 8 millimètres et une longueur comprise entre 15 et 30 centimètres. Ils sont constitués d'un ensemble optique de prise d'images et de transport d'images. Ils ont une bonne résolution mais leur utilisation est nécessairement limitée à des organes particuliers, comme la vessie ou l'exploration des articulations. Ces endoscopes qui comportent un système optique sophistiqué ont un prix assez élevé.

Les endoscopes souples comportent un conducteur d'images constitué d'un paquet de fibres optiques ordonnées. Ils ont un diamètre de quelques millimètres et une longueur qui peut dépasser le mètre permettant d'accéder à des organes tels que les bronches, les intestins ou l'estomac. Ces fibroscopes ont une résolution médiocre nécessairement limitée par le nombre de fibres optiques du conducteur d'images. Ce nombre ne dépasse pas 80 000 pour conserver un diamètre faible et une certaine souplesse. Ils ont également une transmission assez mauvaise dans les basses longueurs d'onde comme le bleu. De plus, ils sont fragiles et comme les endoscopes rigides, leur coût est élevé.

Apparus depuis une dizaine d'années, les vidéoendoscopes constituent une nouvelle génération d'endoscopes permettant l'exploration in vivo d'organes peu accessibles. La spécificité des vidéoendoscopes est que la caméra qui filme l'organe examiné est disposée en bout du conduit souple. II n'y a donc plus nécessité de véhiculer optiquement l'image jusqu'à l'autre extrémité de l'endoscope. Cette nouvelle génération est devenue possible grâce à la très faible taille des photo-détecteurs actuels, généralement de type « CCD ». Ainsi, le format dit 1/4" est très répandu, mais on trouve facilement maintenant des formats dit 1/10", c'est-à-dire que la diagonale du photodétecteur ne dépasse pas quelques millimètres. De plus, ces capteurs ont des résolutions élevées. Les meilleures résolutions sont de l'ordre de 850 000 pixels pour des formats 1/4" ou 1/6", soit dix fois supérieures à celles des fibroscopes traditionnels. De plus, la transmission des signaux est bien supérieure, les aberrations chromatiques étant corrigées. On obtient ainsi des images de bien meilleure qualité. On peut lire avec intérêt à ce sujet l'article intitulé « Nouveautés diagnostiques en endoscopie, T.Ponchon, Gastroenteral Clin.Biol. 2001 ».

L'endoscope étant utilisé à l'intérieur du corps humain, un des problèmes majeurs est la nécessité de stériliser le matériel avant chaque intervention à moins que celui-ci ne soit jetable. Or, il n'est pas possible de stériliser correctement un vidéoendoscope puisqu'il faut porter la matrice CCD à des températures importantes, de l'ordre de 120 degrés et ce, en milieu humide.

Un second problème est que ces endoscopes ne comportent pas de voie de fluorescence permettant d'examiner l'organe malade. L'ajout d'une voie de fluorescence n'est pas nécessairement simple dans la mesure où l'encombrement est strictement limité. II est, par exemple, hors de question d'ajouter une seconde matrice de détection dédiée à la lumière de fluorescence.

Le vidéoendoscope selon l'invention résout ces deux problèmes. En effet, il réunit dans une même tête optique l'ensemble des moyens permettant de réaliser à la fois une image en lumière « blanche » et en imagerie de fluorescence sur un même capteur CCD. D'autre part, la simplicité de la tête optique et le prix peu élevé des éléments qui la composent font que la tête optique peut être à usage unique et jetée après usage, évitant ainsi tout problème de stérilisation. Même dans les applications industrielles ne nécessitant pas de stérilisation, il est clair que le coût peu élevé de la tête optique est un attrait important dans la mesure où il n'est plus nécessaire de ménager l'endoscope ou de pratiquer des opérations de nettoyage fastidieuses.

Plus précisément, l'invention a pour premier objet une tête optique bispectrale pour vidéoendoscope comprenant au moins un capteur photosensible, un objectif associé audit capteur photosensible et des moyens d'éclairage en lumière blanche, **caractérisé en ce que** la tête optique comporte également au moins:
un moyen d'éclairage adapté à émettre une lumière monochromatique à une longueur d'onde prédéterminée,
des moyens de filtrage optique permettant de filtrer ladite longueur d'onde prédéterminée dans une bande spectrale étroite, lesdits moyens de filtrage disposés de façon à filtrer l'image formée par l'objectif sur le capteur photosensible, et
des connecteurs électriques ou optiques reliés aux moyens d'éclairage en lumière blanche, audit moyen d'éclairage et au capteur photosensible et destinés à être couplés respectivement à des moyens d'alimentation et à des moyens d'analyse d'image du vidéoendoscope.

Avantageusement, les moyens d'éclairage comportent des liaisons à fibre optique destinées à être couplées à des sources d'éclairage disposées à l'extérieur de la tête optique, la lumière issue desdites sources d'éclairage étant acheminée à la tête optique par lesdites fibres optiques ou, dans une variante, les moyens d'éclairage comportent des sources d'éclairage disposées dans la tête optique.

Avantageusement, les sources d'éclairage en lumière blanche sont au moins une diode électroluminescente blanche, le capteur photosensible étant un capteur « couleur », c'est-à-dire comportant un filtre dit « RGB » disposé devant les pixels du capteur.

A titre alternatif, les sources d'éclairage en lumière blanche sont au moins un triplet de diodes électroluminescentes émettant dans trois bandes spectrales différentes, le capteur photosensible étant monochrome.

Enfin, les moyens d'éclairage en lumière blanche comportent au moins une diode électroluminescente organique ou OLED.

Avantageusement, le moyen d'éclairage adapté à émettre une lumière monochromatique, comporte au moins une diode électroluminescente organique ou OLED et la longueur d'onde prédéterminée du moyen d'éclairage adapté à émettre une lumière monochromatique vaut environ 690 nanomètres.

Avantageusement, la tête optique comporte deux capteurs photosensibles, optiquement couplés à un second moyen de filtrage spectral, le second moyen de filtrage assurant la séparation spectrale entre d'une part un spectre de lumière de fluorescence issue d'un objet déterminé éclairé par la lumière monochromatique et d'autre part le reste du spectre de la lumière blanche. Avantageusement, le second moyen de filtrage comporte une lame dichroïque.

Dans un mode de réalisation privilégié, les capteurs photosensibles sont disposés orthogonalement l'un à l'autre et symétriquement de part et d'autre de la lame dichroïque.

Dans un autre mode de réalisation privilégié, la tête optique comporte des moyens optiques agencés de façon que les capteurs photosensibles soient disposés parallèlement l'un par rapport à l'autre. La tête optique comporte des moyens optiques agencés de façon que les capteurs photosensibles soient disposés dans le même plan et intégrés sur un substrat commun.

Avantageusement, le ou les capteurs photosensibles sont de type CMOS, au format 1/10".

Avantageusement, avant utilisation, la tête optique est conditionnée dans un emballage stérile jetable à usage unique.

L'invention a pour second objet un vidéoendoscope comprenant une tête optique, une alimentation des moyens d'éclairage de ladite tête optique, des moyens d'analyse et de visualisation d'une image fournie par au moins un capteur photosensible disposé dans ladite tête optique, **caractérisé en ce que**:
la tête optique est telle que définie précédemment ;
en utilisation opérationnelle, les moyens d'éclairage en lumière blanche et en lumière monochromatique, et le ou les capteurs photosensibles sont reliés respectivement à l'alimentation et aux moyens d'analyse d'image du vidéoendoscope par des connecteurs, et
le vidéoendoscope comporte des moyens permettant d'adresser séquentiellement soit les moyens d'éclairage en lumière blanche, soit le moyen d'éclairage adapté à émettre une lumière monochromatique à une longueur d'onde prédéterminée.

Avantageusement, lorsque les moyens d'éclairage en lumière blanche sont au moins un triplet de diodes électroluminescentes émettant dans trois bandes spectrales différentes, le vidéoendoscope comporte des moyens permettant d'adresser séquentiellement chaque diode dudit triplet de diodes électroluminescentes.

L'invention sera mieux comprise et d'autres avantages apparaîtront à la lecture de la description qui va suivre donnée à titre non limitatif et grâce aux figures annexées parmi lesquelles:
La figure 1 représente une vue générale d'une tête optique selon l'invention et de l'ensemble du vidéoendoscope associé dans un premier mode de réalisation ;
La figure 2 représente la variation de transmission en fonction de la longueur d'onde des moyens de filtrage optique;
La figure 3 représente un second mode de réalisation de la tête optique dans une version comportant deux capteurs;
La figure 4 représente une première variante de ce second mode de réalisation de la tête optique;
La figure 5 représente une seconde variante de ce second mode de réalisation de la tête optique;
La figure 6 représente une troisième variante de ce second mode de réalisation de la tête optique.

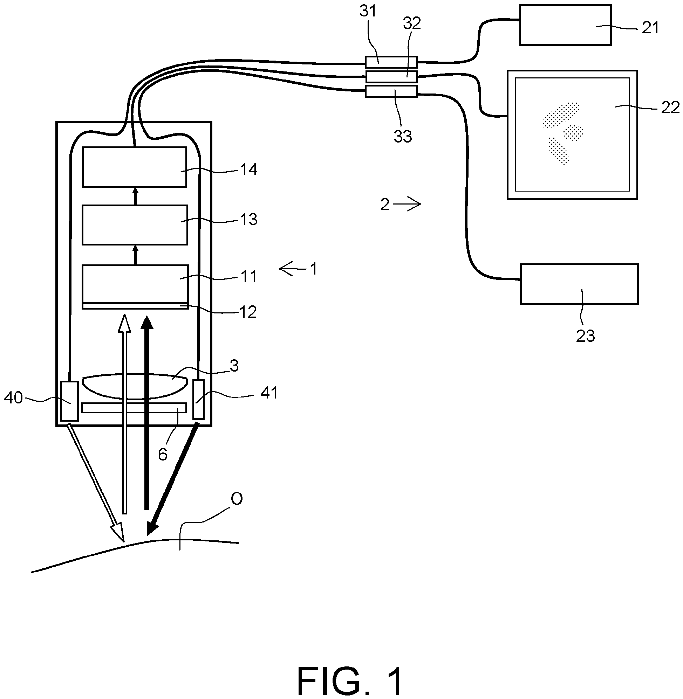

A titre de premier exemple non limitatif, la figure 1 représente une vue générale d'un premier mode de réalisation d'un vidéoendoscope selon l'invention. II comporte essentiellement une tête optique 1 et un ensemble 2 d'alimentation et d'exploitation des images issues de la tête optique 1.

La tête optique 1 comporte une micro-caméra constituée du capteur photosensible 11 et de l'objectif 3. La micro-caméra forme une image de l'objet O sur le capteur 11. Une des exigences importantes de la tête optique, compte-tenu de son utilisation possible en milieu médical, est qu'elle ait un diamètre le plus faible possible. Le capteur photosensible est donc préférentiellement un capteur de type « CMOS » au format 1/10" comportant de l'ordre du million de pixels. Les dimensions du capteur n'excèdent pas quelques millimètres, le champ image est un carré de 2 millimètres de côté et la taille des pixels élémentaires ne dépasse pas 2 microns. L'objectif est une microoptique de focale 2 millimètres. Si cet objectif a un grandissement de 5, il permet de faire une image haute résolution d'un objet dont les dimensions font environ 10x10 millimètres. La distance de travail, c'est-à-dire la distance séparant le dernier dioptre de l'objectif de l'objet à observer est de l'ordre de 12 mm. L'ouverture numérique ON est alors de 0,42. Ce type de capteur et d'optique sont couramment utilisés pour des applications en téléphonie mobile.

Le capteur photosensible est relié à des moyens de formation d'image, par exemple un amplificateur 13 et à un convertisseur analogique numérique 14. La sortie du convertisseur 14 est raccordée à un système classique d'exploitation de l'image 22 au moyen de la connectique 32.

La tête optique possède deux voies d'imagerie.

La première voie permet de réaliser des images couleur de l'objet à observer. Elle comprend des moyens d'éclairage en lumière blanche 40. Le rayonnement lumineux est représenté par une flèche blanche sur la figure 1 et sur les autres figures. Ces moyens sont préférentiellement une ou plusieurs micro-diodes électroluminescentes. Ces diodes sont alimentées par une alimentation 21 à travers la connectique 31. La lumière blanche peut être assurée soit par des diodes dites « blanches » émettant directement dans un spectre couvrant tout le spectre visible, soit par des triplets de diodes émettant respectivement dans les bandes spectrales rouges, vertes et bleues du spectre visible. Dans le premier cas, le capteur est nécessairement un capteur « couleur ». II comporte un filtre mosaïque dit « RGB » 12 pour réaliser une image couleur. Dans le second cas, il est possible d'utiliser un capteur monochrome sans filtre RGB à la condition d'adresser séquentiellement les diodes rouges, vertes et bleues. On enregistre ainsi séquentiellement trois images rouge, verte et bleue permettant de reconstituer l'image couleur d'origine. Dans ce cas, la résolution est meilleure mais la cadence d'acquisition est moins rapide. Ce moyen d'éclairage en lumière blanche 40 peut comporter un ou plusieurs diodes électroluminescentes organiques.

La seconde voie permet de réaliser des images de fluorescence de l'objet observé O. Elle comporte un moyen d'éclairage 41, adapté à émettre une lumière monochromatique représentée par une flèche noire sur la figure 1 et sur les autres figures. Ce moyen d'éclairage 41 peut être une fibre optique adaptée à transmettre la lumière monochromatique issue d'une source d'émission laser fibrée 23. Préférentiellement, cette source émet à une longueur d'onde de 690 nm, longueur d'onde favorable à la fluorescence des tissus vivants.

La fibre optique est une fibre standard de type « Télécom ». Elle est raccordée au moyen d'une connectique optique 33 à la source 23. L'image de fluorescence obtenue est captée par la micro-caméra 11.

Pour obtenir une image de fluorescence correcte, l'éclairement nécessaire de l'objet éclairé doit être de l'ordre de 25 µW/mm². Pour un champ objet de l'ordre de 100 mm², cela donne une puissance d'excitation de 2 à 3 mW. II n'y a aucune difficulté à véhiculer une telle puissance dans des fibres optiques, puissance qui reste largement en dessous des normes de sécurité laser.

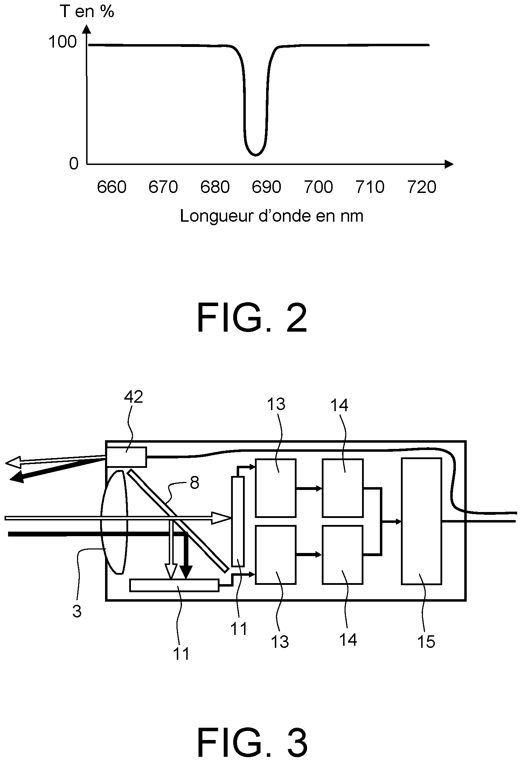

Cette image ne doit pas être polluée par la lumière parasite due à la rétrodiffusion des tissus éclairés par la lumière d'excitation. Pour s'en affranchir, on dispose au niveau de la micro-caméra un filtre 6 dit « notch », c'est-à-dire un filtre interférentiel qui coupe exclusivement la composante spectrale à 690 nm avec une largeur très étroite de l'ordre de quelques nanomètres. A titre indicatif, la transmission de ce filtre en fonction de la longueur d'onde est représentée en figure 2. Ce filtre placé devant l'optique de manière permanente ne dégrade pas sensiblement l'image couleur puisqu'il ne retire du spectre visible qu'une étroite bande de lumière autour de 690 nm, c'est-à-dire que le capteur va restituer intégralement le bleu, le vert et presque tout le rouge de l'image.

L'alternance entre l'image couleur et l'image de fluorescence se fait simplement en allumant séquentiellement les diodes de l'éclairage blanc puis le laser. Le système d'image assure la sélection de l'image à afficher. Ainsi, la micro-caméra est bi-spectrale et permet l'acquisition à la fois d'une image couleur et d'une image de fluorescence. Bien entendu, ces images peuvent être présentées séparément, en superposition et bénéficier de traitements d'image.

La figure 3 montre un second mode de réalisation de la tête optique 1 basé sur l'utilisation de deux capteurs d'image matriciels 11. Les deux capteurs photosensibles sont disposés orthogonalement l'un à l'autre et symétriquement de part et d'autre d'une lame dichroïque 8.

Un des deux capteurs photosensible 11 fonctionne en monochrome encore appelé « noir et blanc ». II permet d'acquérir l'image de fluorescence. Le second capteur photosensible est un capteur couleur. Ces capteurs photosensibles sont reliés à des moyens de formation d'image, par exemple des amplificateurs 13 et des convertisseurs analogique-numérique 14, ces ensembles formant soit une caméra noir et blanc, soit une caméra couleur. De tels capteurs photosensibles peuvent être des capteurs réalisés en technologie CCD ou CMOS. La caméra noir et blanc et la caméra couleur peuvent être reliées à un multiplexeur 15, pour la transmission du signal numérique vers l'unité de réception d'images distante de l'endoscope.

Selon une première variante, le dispositif a deux modes de fonctionnement distincts, pouvant être exécutés séquentiellement pour acquérir l'image de fluorescence et l'image couleur. Durant l'acquisition de l'image de fluorescence, la lumière d'excitation arrive par la fibre optique d'éclairage 42, le spectre de cette lumière correspond au spectre d'absorption du fluorophore du tissu et plus généralement de l'objet que l'on souhaite observer. La lumière de fluorescence est alors captée par l'objectif 3 et est réfléchie par le filtre dichroïque 8. Dans l'exemple de la figure 3, le filtre dichroïque 8 est réfléchissant pour cette gamme de longueur d'onde. II est également possible de disposer un filtre « notch » devant l'objectif 3.

Durant l'acquisition de l'image visible, la lumière envoyée dans la fibre optique d'éclairage 42 est une lumière blanche. Celle-ci se réfléchit sur les tissus et est spectralement divisée par le filtre dichroïque 8. L'image couleur est obtenue par la sommation des images recueillies sur les deux capteurs photosensibles 11.

Selon une seconde variante, ces deux modes coexistent simultanément en envoyant dans la fibre optique une lumière dont le spectre contient l'ensemble du spectre compris entre le bleu et la longueur d'onde à laquelle on souhaite séparer le spectre de fluorescence de celui de l'excitation. Dans ce cas de figure, l'image de fluorescence est obtenue sur un seul des deux capteurs photosensibles 11, et l'image couleur, en sommant les images obtenues par les deux capteurs photosensibles 11.

Bien entendu, il est possible d'utiliser un filtre dichroïque transmettant la lumière de fluorescence au lieu de la réfléchir.

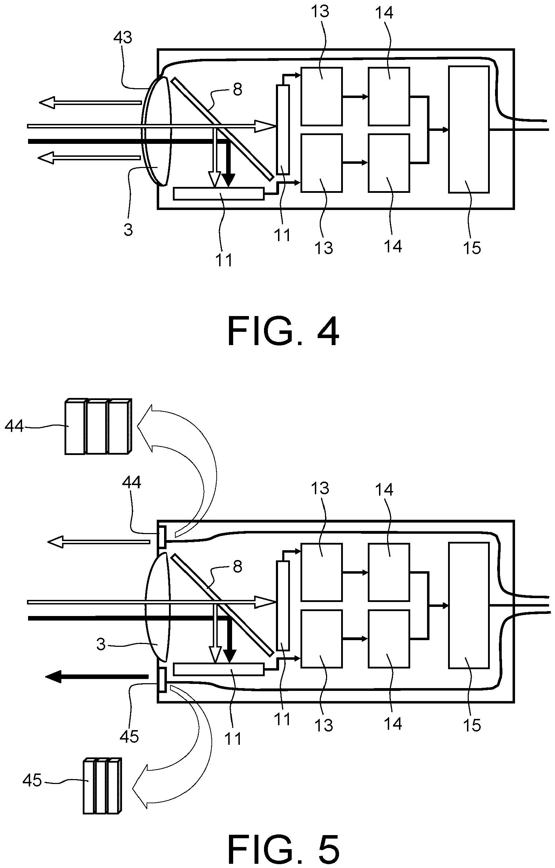

Comme on l'a vu dans les exemples de réalisations précédentes, la lumière issue de la tête optique peut provenir de fibres optiques connectées à des sources de lumière externes à la tête optique. On dispose ainsi de sources lumineuses puissantes et spectralement bien définies. II est également possible, pour éviter l'emploi de fibres optiques, de disposer les sources de lumière à l'intérieur même de la tête optique. La figure 4 représente ainsi une première variante de réalisation de la tête optique telle que représentée en figure 3. Dans cette variante, une source de lumière 43 est disposée en extrémité de tête optique. Les moyens de détection et de filtrage sont similaires à ceux du mode de réalisation précédent. Cette source de lumière est réalisée en déposant un polymère organique sur la surface de l'objectif. Ce polymère est de type OLED pour « Organic Light Emitting Diode » ou encore « Diode Electro-Luminescente Organique ». Ce polymère est choisi pour émettre le spectre situé en dessous de la longueur d'onde de fluorescence afin de ne pas perturber l'acquisition d'image de fluorescence. Une autre possibilité est de déposer deux polymères, l'un émettant le spectre compris entre le début du spectre visible et la longueur d'onde d'excitation incluse, le second polymère émettant le spectre compris entre la longueur d'onde d'excitation et la partie rouge du spectre visible.

En allumant simultanément les deux OLED, on reconstruit une source d'éclairage blanche non distordue. Selon ce mode de réalisation, chaque polymère formant un OLED est relié à une source de tension, non représentée sur la figure 4, laquelle est soit intégrée dans la tête optique de l'endoscope, soit placée à distance et reliée par un moyen de connexion électrique. Cette source de tension peut être commune à celle alimentant les capteurs d'image 11 ou les moyens de formation d'image 13, 14 et 15.

La figure 5 représente une seconde variante de réalisation de la tête optique telle que représentée en figure 3. Elle comporte des caractéristiques de détection similaires à celles du mode de réalisation exposée sur la figure 3 et sur la figure 4. Selon ce mode de réalisation, la source de lumière est embarquée en extrémité d'endoscope sous la forme d'une barrette 44 de diodes électroluminescentes ou LED tricolores, destinées à reconstruire une source de lumière blanche. Cette barrette peut être remplacée par des diodes blanches. Toutefois, l'utilisation de trois LEDs séparées est préférable dans la mesure où il devient possible de régler la température de couleur en fonction du type de pathologie que l'on souhaite observer. Dans ce mode de réalisation, la source de lumière 45 permettant d'exciter la fluorescence est une barrette d'une ou de plusieurs diodes laser à la longueur d'onde d'absorption du fluorophore étudié.

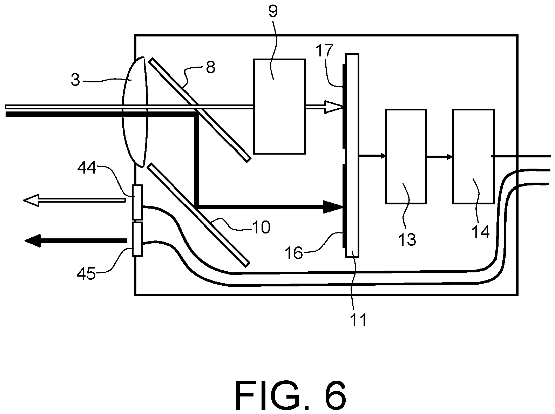

La figure 6 décrit un mode de réalisation dans lequel une seule caméra est utilisée pour réaliser les deux images couleur et de fluorescence. Par caméra, on entend ici un capteur photosensible matriciel, relié à des moyens de formation d'image, ces moyens étant par exemple un amplificateur 13 et un convertisseur analogique numérique 14. Selon ce mode de réalisation, le capteur photosensible matriciel est divisé en deux capteurs photosensibles 16 et 17, chacun occupant une partie de la matrice, chacun étant destiné à acquérir une image différente. L'avantage est un coût réduit et une plus grande miniaturisation. La source de lumière est identique à celle décrite dans le mode de réalisation de la figure 5 toutefois, les LEDs et les diodes laser sont regroupées sous l'objectif de l'endoscope afin de tirer parti de la place laissée libre par la dissymétrie de l'ensemble. La lumière de fluorescence réfléchie sur l'objet étudié est collectée par l'objectif 3, puis réfléchie par une lame dichroïque 8. Elle est alors réfléchie sur un miroir plan 10 puis projetée sur la moitié inférieure 16 du capteur d'image 11. La lumière blanche issue de l'objet et collectée par l'objectif 3 est transmise par la lame dichroïque 8 et filtrée par un filtre de fluorescence. Elle atteint alors la partie supérieure 17 du capteur d'image 11 qui la numérise. Pour équilibrer les deux trajets optiques et éviter que l'une des deux images soit défocalisée par rapport à l'autre, on place une lame de verre 9 dans le trajet optique le plus court de manière que les deux trajets optiques soient les même pour l'image de fluorescence et l'image blanche. II est possible d'achromatiser cette lame en la réalisant dans deux verres d'indice optique et de constringence différents. On rappelle que le trajet optique noté ∂ et est tel que:
∂ = n x d, avec:
n : indice optique traversé
d : distance parcourue pour l'image de fluorescence et l'image blanche.

L'utilisation de micro-caméras dont les composants sont issus de la téléphonie mobile, de micro-diodes électroluminescentes et de fibres optiques standards venant des télécommunications permet d'atteindre des coûts de réalisation de la tête optique très faibles. Ainsi, la tête optique peut être à usage unique et jetée après utilisation. Dans ce cas, elle est conditionnée dans un emballage stérile à usage unique. On évite ainsi tout risque de contamination et on simplifie considérablement l'utilisation du vidéoendoscope en éliminant les complexes opérations de stérilisation après utilisation.

## Revendications

1. Tête optique bispectrale (1) pour vidéoendoscope comprenant au moins un capteur photosensible (11), un objectif (3) associé audit capteur photosensible et des moyens d'éclairage en lumière blanche (40, 42, 43, 44), **caractérisé en ce que** la tête optique comporte également au moins:
un moyen d'éclairage (41, 45) adapté à émettre une lumière monochromatique à une longueur d'onde prédéterminée,
des moyens de filtrage optique (6, 8) permettant de filtrer ladite longueur d'onde prédéterminée dans une bande spectrale étroite, lesdits moyens de filtrage disposés de façon à filtrer l'image formée par l'objectif sur le capteur photosensible, et
des connecteurs électriques ou optiques (31, 32, 33) reliés aux moyens d'éclairage en lumière blanche, audit moyen d'éclairage et au capteur photosensible et destinés à être couplés respectivement à des moyens d'alimentation (21) et à des moyens d'analyse d'image (22) du vidéoendoscope.

2. Tête optique bispectrale pour vidéoendoscope selon la revendication 1, **caractérisé en ce que** les moyens d'éclairage comportent des liaisons à fibres optiques destinées à être couplés à des sources d'éclairage (23) disposées à l'extérieur de la tête optique, la lumière issue desdites sources d'éclairage étant acheminée à la tête optique par lesdites liaisons à fibre optique.

3. Tête optique bispectrale pour vidéoendoscope selon la revendication 1, **caractérisé en ce que** les moyens d'éclairage comportent des sources d'éclairage disposées dans la tête optique.

4. Tête optique bispectrale pour vidéoendoscope selon l'une des revendications 1 à 3, **caractérisé en ce que** les sources d'éclairage en lumière blanche sont au moins une diode électroluminescente blanche, le capteur photosensible étant un capteur « couleur », c'est-à-dire comportant un filtre dit « RGB » (12) disposé devant les pixels du capteur.

5. Tête optique bispectrale pour vidéoendoscope selon l'une des revendications 1 à 3, **caractérisé en ce que** les sources d'éclairage en lumière blanche (44) sont au moins un triplet de diodes électroluminescentes émettant dans trois bandes spectrales différentes, le capteur photosensible étant monochrome.

6. Tête optique bispectrale pour vidéoendoscope selon l'une des revendications 1 à 3, **caractérisé en ce que** les sources d'éclairage en lumière blanche (43) comportent au moins une diode électroluminescente organique ou OLED.

7. Tête optique bispectrale pour vidéoendoscope selon les revendications 1 à 6, **caractérisé en ce que** la source d'éclairage adaptée à émettre une lumière monochromatique (43) comporte au moins une diode électroluminescente organique ou OLED.

8. Tête optique bispectrale pour vidéoendoscope selon les revendications 1 à 7, **caractérisé en ce que** la longueur d'onde prédéterminée du moyen d'éclairage adapté à émettre une lumière monochromatique vaut environ 690 nanomètres.

9. Tête optique bispectrale pour vidéoendoscope selon les revendications 1 à 8, **caractérisé en ce qu'**elle comporte deux capteurs photosensibles (11), optiquement couplés à un second moyen de filtrage spectral (8), le moyen de filtrage assurant la séparation spectrale entre, d'une part un spectre de lumière de fluorescence issue d'un objet déterminé éclairé par la lumière monochromatique et d'autre part le reste du spectre de la lumière blanche.

10. Tête optique bispectrale pour vidéoendoscope selon la revendication 9, **caractérisée en ce que** le second moyen de filtrage comporte une lame dichroïque (8).

11. Tête optique bispectrale pour vidéoendoscope selon la revendication 10, **caractérisé en ce que** les capteurs photosensibles sont disposés orthogonalement l'un à l'autre et symétriquement de part et d'autre de la lame dichroïque.

12. Tête optique bispectrale pour vidéoendoscope selon la revendication 9, **caractérisé en ce que** la tête optique comporte des moyens optiques (10) agencés de façon que les capteurs photosensibles soient disposés parallèlement l'un par rapport à l'autre.

13. Tête optique bispectrale pour vidéoendoscope selon la revendication 12, **caractérisé en ce que** la tête optique comporte des moyens optiques (9) agencés de façon que les capteurs photosensibles (16, 17) soient disposés dans le même plan et intégrés sur un substrat commun (11).

14. Tête optique bispectrale pour vidéoendoscope selon l'une des revendications 1 à 12, **caractérisé en ce que** le ou les capteurs photosensibles sont de type CMOS, au format 1/10".

15. Tête optique bispectrale pour vidéoendoscope selon l'une des revendications précédentes, **caractérisé en ce que**, avant utilisation, la tête optique est conditionnée dans un emballage stérile jetable à usage unique.

16. Vidéoendoscope comprenant une tête optique, une alimentation des moyens d'éclairage de ladite tête optique, des moyens d'analyse et de visualisation d'une image fournie par au moins un capteur photosensible disposé dans ladite tête optique, **caractérisé en ce que**:
la tête optique est selon l'une des revendications précédentes, et
en utilisation opérationnelle, les moyens d'éclairage en lumière blanche et en lumière monochromatique et le ou les capteurs photosensibles sont reliés respectivement à l'alimentation et aux moyens d'analyse d'image du vidéoendoscope par des connecteurs ;
le vidéoendoscope comporte des moyens permettant d'adresser séquentiellement soit les moyens d'éclairage en lumière blanche, soit le moyen d'éclairage adapté à émettre une lumière monochromatique à une longueur d'onde prédéterminée.

17. Vidéoendoscope selon la revendication 16, **caractérisé en ce que**, lorsque les moyens d'éclairage en lumière blanche sont au moins un triplet de diodes électroluminescentes émettant dans trois bandes spectrales différentes, ledit vidéoendoscope comporte des moyens permettant d'adresser séquentiellement chaque diode dudit triplet de diodes électrolum inescentes.
